# EUROPEAN PATENT APPLICATION

(11) **EP 3 711 782 A1**
(43) Date of publication of application: **23.09.2020**
(21) Application number: 18892982.2
(22) Date of filing: 21.12.2018
(51) Int. Cl.: A61L 15/60, A61L 15/22, A61L 15/28, A61L 15/24, A61L 15/42, A61L 15/58

(54) **HYDROCOLLOID COMPOSITION AND BIOPATCH COMPRISING SAME**

(30) Priority: 21.12.2017 KR 20170176901
(71) Applicant: Industry-University Cooperation Foundation Hanyang University, Seoul 04763 (KR)
(72) Inventor: KIM, Tae Won, Seoul 05649 (KR)
(74) Representative: Prüfer & Partner mbB Patentanwälte · Rechtsanwälte
(86) International application number: PCT/KR2018/016452
(87) International publication number: WO 2019/125043

(57) **Abstract**

Hydrocolloid composition and bio-patch comprising the same are provided. The hydrocolloid composition has a hydrophobic elastomer matrix. Hydrocolloid particles and aerogel particles are dispersed in the matrix. The aerogel particles may be silica aerogel particles. The aerogel particles may include particles having hydrophobic functional groups formed on the particle surface. The aerogel particles may be hydrophobic aerogel particles, hybrid aerogel particles in which hydrophobic functional groups and hydrophilic functional groups are simultaneously formed on the particle surface, or a mixture of the hydrophobic aerogel particles, the hybrid aerogel particles, and hydrophilic aerogel particles.

## Description

### [Technical Field]

The present embodiment relates to a bio-patch, and more particularly, to a bio-patch in the form of hydrocolloid.

### [Background Art]

Methods of protecting and treating wounds, including drug treatment, are referred to as dressing, and the dressing can be divided into a dry environment gauze dressing and a closed wet dressing. In the case of the dry environment gauze dressing, moisture is evaporated and removed through the gauze, so there is a disadvantage in that a crust is formed on the wound to prevent the regenerated epithelial cells from moving smoothly along the wound surface, leaving a scar. In contrast, in the case of the closed wet dressing, the wound is prevented from drying out and the wound is placed in a wet state to smoothly restore the wound.

Closed wet dressings can be classified as semi-permeable film dressings, foam dressings, or hydrocolloid dressings, which can be used separately depending on the amount of exudate from the wound. In hydrocolloid dressings, hydrophilic molecules absorb exudate from the wound, but there is a limit to the extent of exudate absorption, so the dressing needs to be changed frequently if a large amount of exudate occurs. In addition, the existing hydrocolloid dressing may have a problem that may cause pain and odor due to delayed wound healing as the moisture permeability and waterproof function are still insufficient.

### [Disclosure]

### [Technical Problem]

The problem to be solved by the present invention is to provide a hydrocolloid-type bio-patch that can insulate heat.

Another problem to be solved by the present invention is to provide a hydrocolloid-type bio-patch with improved moisture permeability and water resistance.

Another problem to be solved by the present invention is to provide a hydrocolloid-type bio-patch with improved exudate absorption capacity.

### [Technical Solution]

One aspect of the present invention provides a hydrocolloid composition. The hydrocolloid composition comprises a hydrophobic elastomer matrix. A plurality of hydrocolloid particles and a plurality of aerogel particles are dispersed in the matrix. The hydrocolloid composition may contain 100 parts by weight of the hydrophobic elastomer, 70 to 150 parts by weight of the hydrocolloid particles, and 1 to 15 parts by weight of the aerogel particles.

The hydrophobic elastomer may be a styrene-isoprene-styrene triblock copolymer (SIS). The hydrocolloid particles may be alginate crosslinked by calcium ions.

The aerogel particles may be silica aerogel particles. The aerogel particles may have particles with hydrophobic functional groups formed on the particle surface. The aerogel particles may be hydrophobic aerogel particles, hybrid aerogel particles in which hydrophobic functional groups and hydrophilic functional groups are simultaneously formed on the particle surface, or a mixture of the hydrophobic aerogel particles, the hybrid aerogel particles and hydrophilic aerogel particles.

The hydrocolloid composition may further comprise an adhesive and/or a plasticizer.

Another aspect of the present invention provides a bio-patch. The bio-patch comprises a hydrocolloid sheet having a hydrophobic elastomer matrix, and hydrocolloid particles and aerogel particles dispersed within the matrix. The bio-patch may further comprise an adhesive layer which is a hydrophilic adhesive layer disposed on the upper surface of the hydrocolloid sheet; and/or a base layer that is a moisture-permeable and water-repellent film disposed on the lower surface of the hydrocolloid sheet. The hydrocolloid sheet may contain 100 parts by weight of the hydrophobic elastomer, 70 to 150 parts by weight of the hydrocolloid particles, and 1 to 15 parts by weight of the aerogel particles. The hydrocolloid sheet may further comprise an adhesive and/or a plasticizer.

### [Advantageous Effects]

According to the present invention as described above, it is possible to provide a hydrocolloid-type bio-patch that insulates heat as it contains a highly porous aerogel. In addition, it is possible to improve the moisture permeability and waterproof function of the hydrocolloid-type bio-patch. In addition to this, it is possible to improve the ability of the hydrocolloid-type bio-patch to absorb exudate.

However, the effects of the present invention are not limited to the above-mentioned effects, and other effects not mentioned will be clearly understood by those skilled in the art from the following description.

### [Description of Drawings]

FIG. 1 is a schematic view showing a cross-section of a bio-patch according to an embodiment of the present invention.
FIG. 2 is a schematic view showing a state in which the bio-patch according to an embodiment of the present invention is applied to a wound site.
FIGS. 3A to 3C are graphs illustrating measurement results of the powders obtained in Aerogel Preparation Examples 1 to 3, respectively, through Fourier-transform infrared spectroscopy (FT-IR spectroscopy).
FIG. 4 is an image illustrating the floating degree of aerogel powder (a) including hybrid aerogel according to Aerogel Preparation Example 2 and a mixture powder (b) of hydrophilic aerogel according to Aerogel Preparation Example 3 and hydrophobic aerogel according to Aerogel Preparation Example 1 in water.
FIG. 5 is a photograph of a bio-patch according to Bio-Patch Preparation Example 1.
FIG. 6 is a graph showing the adhesive strength of the bio-patch according to Bio-Patch Preparation Example 1.
FIG. 7 is a photograph showing the degree of breathability of the bio-patch according to Bio-Patch Preparation Example 1.

### [Modes of the Invention]

Hereinafter, exemplary embodiments of the present invention will be described in detail with reference to the accompanying drawings in order to describe the present invention in more detail. However, the invention is not limited to the embodiments described herein but may be embodied in other forms.

In the present specification, when it is described as "Cx to Cy", it should be construed that the case having the number of carbon atoms corresponding to all integers between carbon number X and carbon number Y also described. In the present specification, when "X to Y" is described, the number corresponding to all integers between X and Y should be interpreted as being described together.

In the present specification, "aerogel" may mean a gel in which the dispersion medium is gas specifically air, and may be a concept including an aerogel in the narrow sense and a xerogel.

### Hydrocolloid composition

The hydrocolloid composition according to an embodiment of the present invention includes a hydrophobic elastomer matrix, and hydrocolloid particles and aerogel particles dispersed in the matrix. The hydrocolloid composition may include 100 parts by weight of the hydrophobic elastomer, 70 to 150 parts by weight of the hydrocolloid particles, and 1 to 15 parts by weight of the aerogel particles, but is not limited thereto and may be variously selected according to the use of the composition.

The hydrophobic elastomer may be natural rubber, polyisoprene, polybutadiene, butyl rubber (e.g., isoprene and isobutylene copolymer), or halogenated butyl rubber, but is not limited thereto. Specifically, the hydrophobic elastomer may be styrene-isoprene-styrene triblock copolymer (SIS) or a mixture of polyisobutylene and SIS.

The hydrocolloid particles may be particles having a network structure in which a hydrophilic polymer forms a three-dimensional crosslink, and may exhibit high water-holding capacity. The three-dimensional crosslink may be formed by physical bonding, for example, hydrogen bonding, van der Waals forces, or hydrophobic interactions; or chemical bonding such as covalent bonding. The hydrophilic polymer constituting these hydrocolloid particles may be a natural hydrophilic polymer, for example, pectin, gelatin, cellulose, specifically, carboxymethylcellulose (CMC), collagen, dextran, elastin, chitin, chitosan, sodium alginate; or a synthetic hydrophilic polymer such as polyacrylic acid (PAA), polyvinyl alcohol, polyethylene glycol, polyvinyl pyrrolidone, polyurethane, polyhydroxyethyl methacrylate, silicone; or combinations thereof. The hydrocolloid particles may further include a crosslinking agent for forming the hydrophilic polymer into a three-dimensional network structure, wherein the crosslinking agent may be calcium chloride, calcium sulfate, calcium nitrate, zinc nitrate, zinc chloride, zinc sulfate, ammonium persulfate, or glutaraldehyde. The crosslinking agent is not limited thereto, and may also vary depending on the type of the hydrophilic polymer. In one example, the hydrocolloid particles may be alginate cross-linked by calcium ions.

The description of the aerogel particles will be described later.

The hydrocolloid composition may further include a pressure-sensitive adhesive and/or a plasticizer. The pressure-sensitive adhesive may be an elastomeric pressure-sensitive adhesive such as polyisobutylene; a phenol-modified terpene; rosin esters, for example glycerol esters of rosin and pentaerythritol esters of rosin, a non-elastomeric pressure-sensitive adhesive including synthetic polyterpene adhesives, or combinations of two or more of these. The plasticizer can be used to provide wetting action and/or viscosity control. These plasticizers are well known in the art and include oil mixtures, waxes, or liquid or soft adhesives, including hydrocarbon oils, liquid hydrocarbon resins, liquid polyterpenes, liquid poly (isobutylene), such as GLISSOPAL, etc. As an example, the plasticizer may be paraffin wax.

The hydrocolloid composition of the present invention may further include a trace amount of other ingredients, for example, pharmacologically active agents such as wound healing promoters, antimicrobial agents, fragrances, deodorants, and, antioxidants, as well known in the art.

### Aerogel particles

The aerogel particles may be high-porous nanostructures having nanometer-sized pores obtained by replacing liquid in a gel structure with air, and may be silica aerogel particles.

The aerogel particles may have a size of 0.1 to 1000 µm, specifically, several to several tens of µm, for example, 1 to 40 µm. The surface area of aerogel particles under the BET method may be 300 to 2000 m²/g, specifically 500 to 1000 m²/g, the density of aerogel particles may be 0.03 to 0.5 g/cc, and the porosity of aerogel particles may be 70 to 99%, and the pore size of aerogel particles may be 5 to 50 nm.

The aerogel particles may have particles with hydrophobic functional groups formed on the particle surface. The hydrophobic functional groups can be disposed not only on the particle surface, but also on the surface of the pores inside the particle. The hydrophobic functional group may be a functional group bonded to an atom constituting the aerogel particle, for example, a functional group bonded to Si atom, and may be hydrogen, a C1-C18 linear or branched alkyl group, a silyloxy group represented by Chemical Formula 1 below, or a combination thereof.

[Chemical Formula 1] *-OSiH₍₃₋ₙ₎R¹ₙ

In Chemical Formula 1, R¹ may be a C1-C18 linear or branched alkyl group, n may be an integer from 0 to 3, and * may represent a bond connected to Si in the aerogel particle.

When n is an integer of 1 to 3, the functional group represented by Formula 1 may be referred to as an alkylsilyloxy group. The C1-C18 linear or branched alkyl group may be a C1-C6 linear alkyl group, and the C1-C6 linear alkyl group may be a saturated linear alkyl group, for example, methyl, ethyl, n-propyl, n-butyl , n-pentyl, or n-hexyl. In one embodiment, the C1-C6 linear alkyl group may be a methyl group or an ethyl group.

In one example, the aerogel particles may be hydrophobic aerogel particles having 80 mol% to 100 mol% specifically 85 mol%, 90 mol%, 95 mol%, or even 99 mol% or more of hydrophobic functional groups based on total functional groups on a particle surface. The particle surface may further include a particle inner pore surface.

In another example, the aerogel particles may be hybrid aerogel particles having hydrophilic functional groups as well as hydrophobic functional groups on the particle surface. The particle surface may further include the surface of interior pores of the particle.

In still another example, the aerogel particles may be a mixture of the hybrid aerogel particles, the hydrophobic aerogel particles, and hydrophilic aerogel particles. Here, the hydrophilic aerogel particles may have 80 mol% to 100 mol% specifically 85 mol%, 90 mol%, 95 mol%, or even 99 mol% or more of hydrophilic functional groups based on total functional groups on a particle surface. The particle surface may further include a particle inner pore surface. The hydrophilic functional group may be bonded to an atom constituting the aerogel particle, for example, be bonded to Si atom, and may be a hydroxyl group (-OH). This mixture may contain the hybrid aerogel particles at about 25 to 40 wt%, the hydrophilic aerogel particles at about 25 to 40 wt%, and the hydrophobic aerogel particles at about 25 to 40 wt%. In one embodiment, the hybrid aerogel particles, the hydrophilic aerogel particles, and the hydrophobic aerogel particles may be contained in a weight ratio of 1: 1: 1.

The hybrid aerogel particles may have the hydrophobic functional groups and the hydrophilic functional groups on their surfaces in a molar ratio of 7: 3 to 3: 7.

Meanwhile, the hybrid aerogel particles, the hydrophilic aerogel particles, and the hydrophobic aerogel particles may be classified according to affinity for water. Specifically, when the aerogel particles are placed in a container with water, the hydrophilic aerogel particles may sink to the bottom of the container, the hydrophobic aerogel particles may float on the water surface, and the hybrid aerogel particles may be half submerged or submerged just below the water surface.

The method of manufacturing aerogel powder having the hybrid aerogel particles may be as follows. However, it is not limited thereto. First, a hydrophobic aerogel powder may be prepared, and the hydrophobic aerogel powder may be heat-treated to partially modify the outer surface and further the surface of the inner pores of the particles provided in the powder. Specifically, the hydrophobic surface functional groups of at least some of the particles provided in the hydrophobic aerogel powder may be changed to hydrophilic surface functional groups. Specifically, the hydrophobic surface functional groups such as hydrogen, an alkyl group or a silyloxy group, specifically an alkylsilyloxy group, can be converted to a hydroxyl group by heat treatment. At the same time, residual moisture in the aerogel powder can be at least partially or completely removed. The heat treatment of the hydrophobic aerogel powder may include a temperature increase step of gradually raising the temperature of the hydrophobic aerogel powder, and a sintering step of leaving the hydrophobic aerogel powder in a heated state for a predetermined time to sinter.

Accordingly, as described above, at least some of the particles of the aerogel powder may be converted into hybrid aerogel particles having both a hydrophobic surface and a hydrophilic surface, and some other particles may be converted into hydrophilic aerogel particles having a hydrophilic surface, and some other particles may remain as hydrophobic aerogel particles that maintain a hydrophobic surface. As a result, it is possible to obtain an aerogel powder that is a mixture containing all of hydrophilic aerogel particles, hydrophobic aerogel particles, and hybrid aerogel particles.

The heat treatment may be performed using an electric furnace, and may be performed for 0.5 to 24 hours in a state where the temperature is raised to a temperature of 300 to 500 °C. In addition, the heat treatment may be performed in an oxidizing atmosphere, specifically, an air atmosphere. When the heat treatment temperature or time is adjusted, only some of the hydrophobic surface functional groups of the hydrophobic aerogel can be changed to hydrophilic surface functional groups, or almost all of the hydrophobic surface functional groups of the hydrophobic aerogel can be changed into hydrophilic surface functional groups to form hydrophilic aerogel. As an example, changing only some hydrophobic surface functional groups of the hydrophobic aerogel to hydrophilic surface functional groups may be performed through heat treatment of about 345 to 355 °C, specifically 347 to 353 °C, and forming hydrophilic aerogel in which all of the hydrophobic surface functional groups of the hydrophobic aerogel has been changed into hydrophilic surface functional groups may be performed through heat treatment of about 356 to 365 °C, specifically 357 to 363 °C.

### Bio-patch

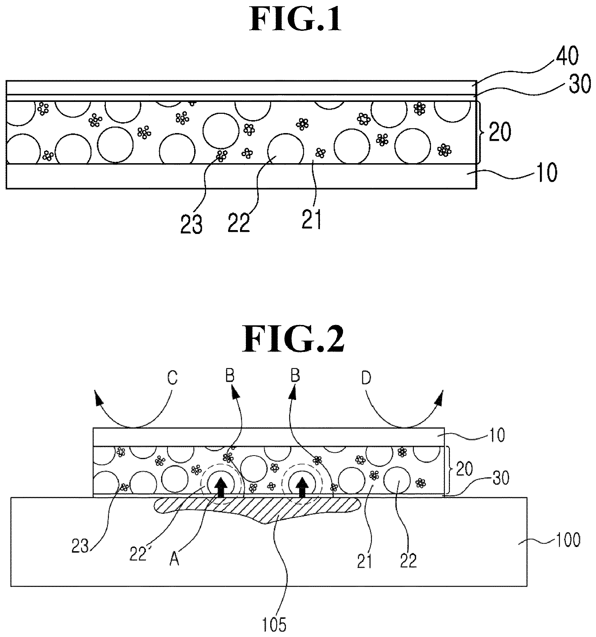

FIG. 1 is a schematic view showing a cross-section of a bio-patch according to an embodiment of the present invention.

Referring to FIG. 1, a bio-patch may include a hydrocolloid sheet 20, an adhesive layer 30, and a release layer 40 on a base layer 10. However, at least one of the base layer 10, the adhesive layer 30, and the release layer 40 may be omitted.

The base layer 10 may be a moisture-permeable water-repellent film, and may be a porous polyethylene or polyurethane film. The base layer 10 may block water droplets and external pathogens, such as bacteria, having a larger size than micropores in the layer (water-repellent properties), while transmitting water vapor and air through the micropores (moisture-permeable properties). The base layer 10 may have a thickness of about 10 to 100 µm.

The hydrocolloid sheet 20 may be formed by laminating the hydrocolloid composition described above on the base layer 10. Specifically, after extruding the hydrocolloid composition on the base layer 10, it can be formed into a layer having uniform thickness using a heating press, a roller, or the like, thereby forming the hydrocolloid sheet 20.

The hydrocolloid sheet 20 may include a hydrophobic elastomer matrix 21. Hydrocolloid particles 22 and aerogel particles 23 may be dispersed in the hydrophobic elastomer matrix 21. Meanwhile, during the preparation of the hydrocolloid composition, through the process of physically and homogeneously blending the hydrophobic elastomer, hydrocolloid particles, and aerogel particles, the hydrocolloid particles and the aerogel particles can be almost homogeneously mixed in the hydrophobic elastomer matrix. Accordingly, the hydrocolloid sheet 20 may include hydrocolloid particles 22 and aerogel particles 23 homogeneously dispersed in the hydrophobic elastomer matrix 21.

The adhesive layer 30 is a layer that may provide adhesion so that the hydrocolloid sheet 20 can be in close contact with the skin. The adhesive layer 30 may be a pressure-sensitive adhesive layer specifically, a hydrophilic pressure-sensitive adhesive layer. The adhesive layer 30 may be, for example, a rosin ester layer, a silicone layer, or a composite layer thereof. However, the present invention is not limited thereto, and various materials used in the art may be used. The adhesive layer 30 may be formed to be very thin with a thickness of several µm.

The release layer 40 is a layer that may be removed immediately before attaching the bio-patch to the wounded skin, and may be formed using a layer widely used in the art.

FIG. 2 is a schematic view showing a state in which the bio-patch according to an embodiment of the present invention is applied to a wound site.

Referring to FIG. 2, the bio-patch is applied on the skin 100 where the wound 105 has been occurred. The release layer 40 described with reference to FIG. 1 may be removed from the bio-patch and adhesive layer 30 may be attached to the skin 100 including the wound 105. When the adhesive layer 30 is omitted, the hydrocolloid sheet 20 may be arranged to directly contact the wound.

The base layer 10, which is a film exhibiting water repellency and moisture permeability, can transmit excess moisture (B) from sweat from the skin 100 or exudate from the wound 105, and remove odor by air permeation, and suppress infiltration of water droplets (C) and pathogens (D) from the outside.

As the aerogel particles 23 dispersed in the hydrocolloid sheet 20 have a high porosity structure, they may contain air in the particles even in the hydrocolloid sheet 20, and thus may exhibit thermal insulation properties. The thermal insulation property of the aerogel particles 23 can maintain the temperature of the wound site at a suitable temperature or higher, thereby improving the speed of wound healing.

In addition, air and water vapor can be transmitted through the aerogel particles 23 having such a high porosity structure, and thus the air permeability and moisture permeability of the hydrocolloid sheet 20 can be improved. As a result, excess moisture (B) of sweat from the skin 100 or exudate from the wound 105 can be emitted to the outside through the hydrocolloid sheet 20 and the base layer 10, thereby causing the wound site 105 can be kept clean and the smell can be reduced.

However, the porous structure of the aerogel particles 23 is not large enough to penetrate water droplets or pathogens. In addition, as the hydrocolloid sheet 20 is provided with a hydrophobic elastomer matrix 21, it may have water-repellent or waterproof properties. Meanwhile, when the aerogel particles 23 have hydrophobic functional groups on the surface thereof, the water repellent or waterproof property of the hydrocolloid sheet 20 may be improved. In this case, it may be considered to omit the base layer 10.

The aerogel particles 23 may be, in one example, hydrophobic aerogel particles having hydrophobic functional groups on most of the particle surface, and in another example, may be hybrid aerogel particles having hydrophilic functional groups as well as hydrophobic functional groups on the particle surface. In another example, the aerogel particles 23 may be a mixture of hydrophobic aerogel particles, hybrid aerogel particles, and hydrophilic aerogel particles.

When the aerogel particles 23 includes hydrophobic functional groups on the surface, due to the hydrophobic functional groups on the surface, these aerogel particles 23 may not be disposed close to the hydrocolloid particles 22 and may be disposed at a certain distance from the hydrocolloid particles 22 exhibiting hydrophilicity. This distance or gap can be a passage through which excess moisture (B) derived from sweat from the skin 100 or exudate from the wound 105 can pass. In addition, the distance or gap can provide a free space for swelling of the hydrocolloid particles. The swelling of the hydrocolloid particles 22 may be occurred when the hydrocolloid particles 22 absorb exudate from the wound 105. In this case, even when the content of the hydrocolloid particles 22 is increased in the hydrocolloid sheet 20, a free space in which the hydrocolloid particles 22 can sufficiently swell can be provided, so that the absorption rate of the hydrocolloid sheet 20 can be greatly increased. This can solve the problem that the conventional hydrocolloid type dressing cannot be used when there are too much exudate from the wound due to the limited absorption rate. In other words, the bio-patch according to this embodiment can be used not only for light wounds with little exudate but also for deep and large wounds with lots of exudate.

When the aerogel particles 23 have hydrophilic functional groups on the surface, that is, the aerogel particles 23 are hybrid aerogel particles or a mixture of hydrophobic aerogel particles, hybrid aerogel particles, and hydrophilic aerogel particles, hydrophilic functional groups of the aerogel particles 23 can provide hydrogen bonding to water vapor, thereby improving moisture permeability of the hydrocolloid sheet 20. In addition, the aerogel particles 23 having hydrophilic functional groups on the surface may also exhibit absorbency for exudate.

Hereinafter, preferred examples are provided to aid the understanding of the present invention. However, the following experimental example is only for helping understanding of the present invention, and the present invention is not limited by the following experimental example.

### <Aerogel Preparation Example 1>

After mixing 2000 g of water with 500 g of sodium silicate solution (SiO₂ 6 wt%) and stirring at a rate of 300 rpm using an electric stirrer, 200 ml of acetic acid was added thereto and mixed for approximately 30 minutes at a rate of 600 rpm using an electric stirrer to prepare a silicate solution. After degassing the silicate solution for about 10 minutes in a vacuum, it was filled in a rectangular frame made of PVC and then subjected to a gelation reaction for 1 hour to prepare silica gel. The silica gel was washed sequentially with water vapor and alcohol to convert the silica gel to alcohol gel, and then the surface of the alcohol gel was treated with a mixed solution of hexane and trimethylchlorosilane (TMCS) to obtain a silylated gel including trimethylsilyloxy group (-OSi(CH₃)₃) on the surface. The obtained silylated gel was dried under normal pressure at a temperature of about 55 °C for about 15 hours, and sintered at a temperature of about 230 °C for about 10 hours to obtain a hydrophobic aerogel. Thereafter, the hydrophobic aerogel was pulverized into a powder to obtain a hydrophobic aerogel powder.

### <Aerogel Preparation Example 2>

The hydrophobic aerogel powder obtained from Aerogel Preparation Example 1 was placed in an electric furnace in an oxidizing atmosphere and heated to 340 °C, followed by sintering for 1 hour while maintaining this temperature.

### <Aerogel Preparation Example 3>

The hydrophobic aerogel powder obtained from Aerogel Preparation Example 1 was placed in an electric furnace in an oxidizing atmosphere and heated to 350 °C, followed by sintering for 1 hour while maintaining this temperature.

### <Aerogel Preparation Example 4>

The hydrophobic aerogel powder obtained from Aerogel Preparation Example 1 was placed in an electric furnace in an oxidizing atmosphere and heated to 360 °C, followed by sintering for 1 hour while maintaining this temperature.

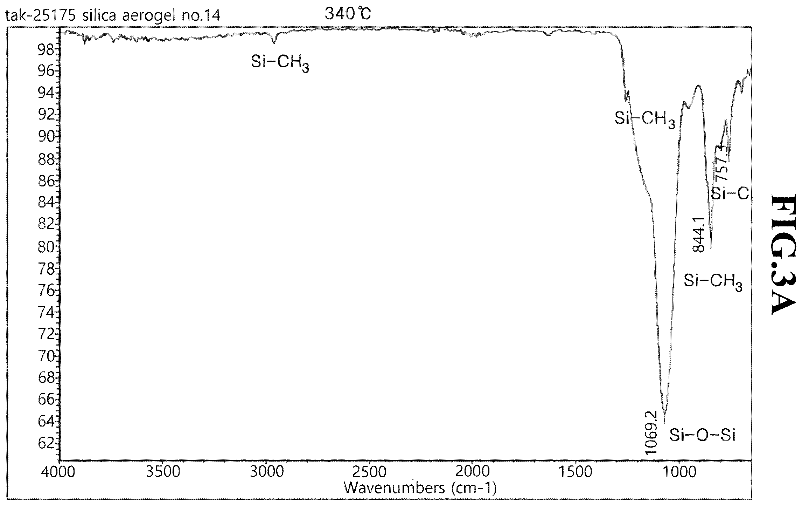

FIGS. 3A to 3C are graphs illustrating measurement results of the powders obtained in Aerogel Preparation Examples 2 to 4, respectively, through Fourier-transform infrared spectroscopy (FT-IR spectroscopy).

Referring to FIG. 3A, it can be seen that the aerogel according to Aerogel Preparation Example 2 shows a Si-CH₃ group in addition to a Si-O-Si group inherent in silica, indicating that the aerogel is hydrophobic aerogel having a hydrophobic surface.

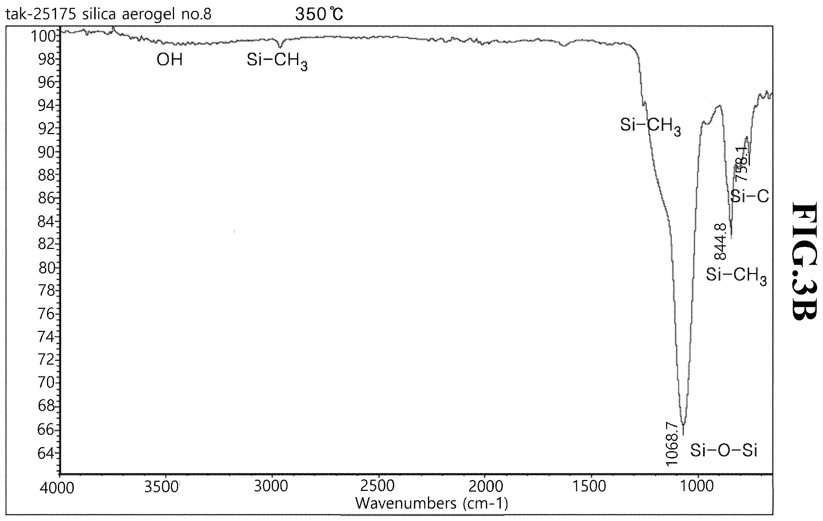

Referring to FIG. 3B, it can be seen that the aerogel according to Aerogel Preparation Example 3 shows both a Si-CH₃ group and an OH group in addition to a Si-O-Si group inherent in silica, indicating that the aerogel is hybrid aerogel formed by partially modifying the hydrophobic surface into a hydrophilic surface through thermal treatment.

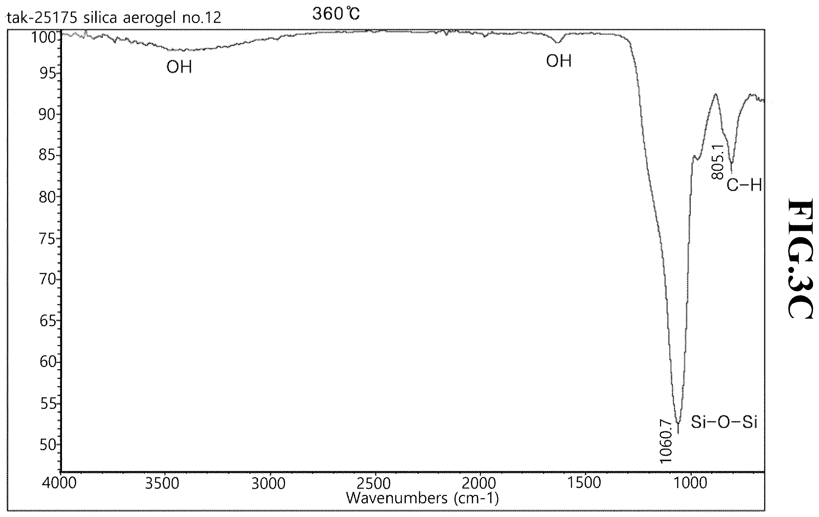

Referring to FIG. 3C, it can be seen that the aerogel according to Aerogel Preparation Example 4 shows an OH group in addition to a Si-O-Si group inherent in silica, and that a peak corresponding to a Si-CH₃ group, which has been observed before surface modification, completely disappears, indicating that the aerogel is converted into hydrophilic aerogel formed by completely modifying the hydrophobic surface into a hydrophilic surface through thermal treatment.

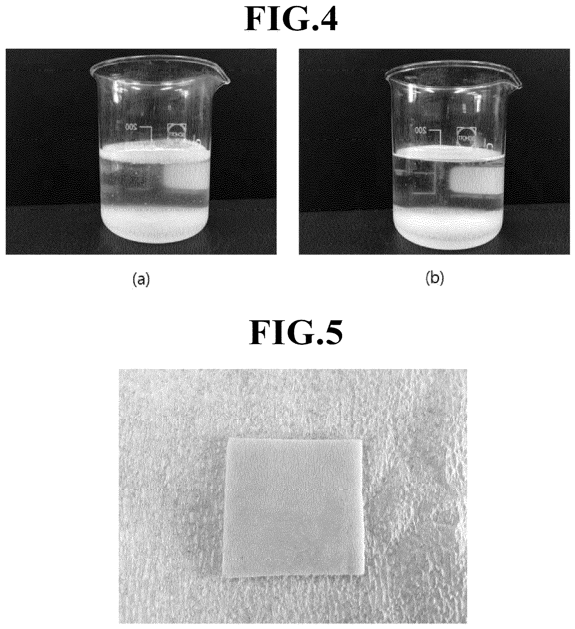

FIG. 4 is an image illustrating the floating degree of aerogel powder including hybrid aerogel according to Aerogel Preparation Example 3 and a mixture of hydrophilic aerogel according to Aerogel Preparation Example 4 and hydrophobic aerogel according to Aerogel Preparation Example 2 in water.

Referring to FIG. 4, it can be seen that, in the case of a mixture (b) of hydrophilic aerogel according to Aerogel Preparation Example 4 and hydrophobic aerogel according to Aerogel Preparation Example 2, hydrophobic aerogel floating on the surface of water and hydrophilic aerogel settled in the bottom of the beaker are clearly distinguished. On the other hand, it can be seen that, in the case of aerogel powder (a) including aerogel according to Aerogel Preparation Example 3, aerogel particles include all of the hybrid aerogel particles approximately half-submerged beneath the surface of water, hydrophilic aerogel particles settled in the bottom of the beaker, and hydrophobic aerogel particles floating on the surface of water.

In addition, simultaneously referring to FIG. 3B and FIG. 4, it can be assumed that the aerogel powder according to Aerogel Preparation Example 3 includes hybrid aerogel having both a Si-CH₃ group and an OH group on a surface thereof, hydrophilic aerogel mainly having an OH group on a surface thereof, and hydrophobic aerogel mainly having a Si-CH₃ group on a surface thereof.

Additionally, each of OH peak intensities in FIGS. 3B and 3C and results of FIG. 4 collectively suggest that the aerogel powder according to Aerogel Preparation Example 3 may include 25 to 40 wt% of the hybrid aerogel particles, 25 to 40 wt% of the hydrophobic aerogel particles, and 25 to 40 wt% of the hydrophilic aerogel particles, and further suggest that these particles are mixed in a weight ratio of 1:1:1.

### <Bio-Patch Preparation Example 1>

2.5 kg of styrene-isoprene-styrene copolymer (SIS) was heated to 170 °C and the temperature was maintained for 20 minutes to liquefy. The liquefied styrene-isoprene-styrene copolymer was cooled to 120 °C, and 2 kg of polyisobutylene and 1.2 kg of liquid paraffin were added thereto, followed by homogeneous mixing to prepare an elastomer mixture. The elastomer mixture was cooled to 90 °C, and 4.3 kg of sodium alginate, 100 g of calcium chloride, and 0.4 kg of hybrid aerogel powder according to Aerogel Preparation Example 3 were added to the cooled mixture, followed by homogeneous mixing to prepare a hydrocolloid-elastomer mixture. The hydrocolloid mixture was extruded at 100 °C to prepare a nanoporous bio-patch.

### <Bio-Patch Preparation Example 2>

Nanoporous bio-patch was prepared in the same manner as in Bio-Patch Preparation Example 1, except that 50 g of calcium chloride and 40 g of hydrophobic aerogel powder according to Aerogel Preparation Example 1 were used.

FIG. 5 is a photograph of a bio-patch according to Bio-Patch Preparation Example 1.

Referring to FIG. 5, it can be seen that a light yellow bio-patch was formed.

### <Bio-patch adhesion test example>

The bio-patch adhesion was measured using the method of ASTM D3330 at a test evaluation institution, and was measured under the conditions of a load cell of 20N, a test speed of 300 MM/MIN, and an adherent SUS 304 using a Universal Testing Machine.

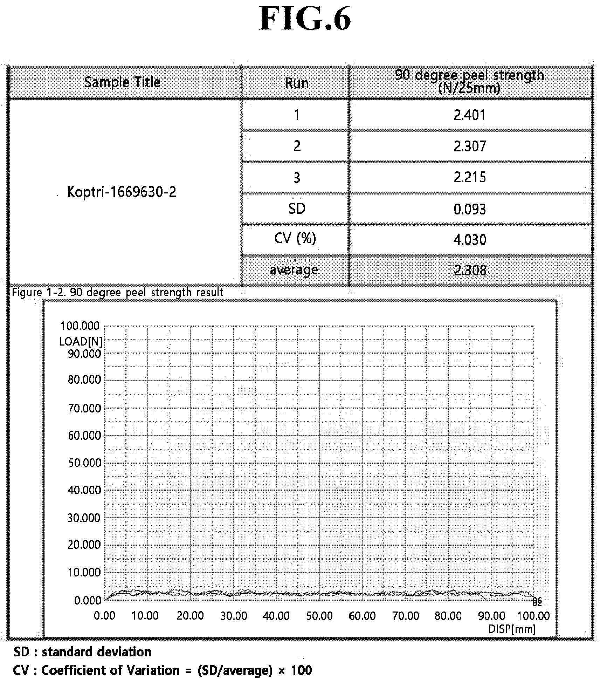

FIG. 6 is a graph showing the adhesive strength of the bio-patch according to Bio-Patch Preparation Example 1.

Referring to FIG. 6, the bio-patch according to the Bio-Patch Preparation Example 1 exhibited an average of 2.31 N/25 mm pressure-sentive adhesion.

### <Bio-patch moisture permeability and absorption test example>

The moisture permeability of the bio-patch was measured according to the EN 13726-2 standard, and the absorbance was measured according to the EN 13726-1 standard.

Table 1 below is a table comparing the performance of the bio-patch according to Bio-Patch Preparation Example 2 and a hydrocolloid dressing product. Here, the hydrocolloid dressing product did not contain aerogel.

**Table 1**

| | bio-patch according to Bio-Patch Preparation Example 2 | hydrocolloid dressing |
|---|---|---|
| moisture permeability | 307 g/m²h | 60 g/m²h |
| exudate absorption rate | 660 % | 112% |
| thickness | 0.8 mm | 0.5 mm |

Referring to Table 1, the bio-patch according to Bio-Patch Preparation Example 2, when compared to the hydrocolloid dressing that does not contain aerogel, has improved moisture permeability by about 5 times despite the increased thickness, and has increased exudate absorption rate by about 6 times.

### <Bio-patch breathability test example>

After placing the bio-patch in a tester, it was examined whether air is permeated through the bio-patch to the top of the bio-patch while introducing air at a constant pressure under the bio-patch.

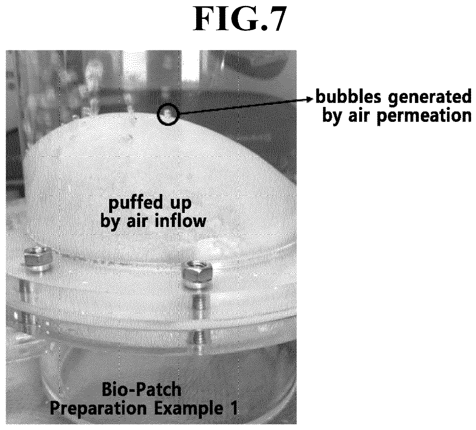

FIG. 7 is a photograph showing the degree of breathability of the bio-patch according to Bio-Patch Preparation Example 1.

Referring to FIG. 7, the bio-patch according to Bio-Patch Preparation Example 1 was swollen by air inflow, and it was confirmed that the air permeability was very excellent because a large amount of bubbles were generated at the top of the bio-patch.

In the above, the present invention has been described in detail with reference to preferred embodiments, but the present invention is not limited to the above embodiments, and various modifications and changes by those skilled in the art is possible within the spirit and scope of the present invention.

## Claims

1. A hydrocolloid composition comprising:
a hydrophobic elastomer matrix; and
hydrocolloid particles and aerogel particles dispersed in the matrix.

2. The hydrocolloid composition according to claim 1, wherein the hydrocolloid composition contains 100 parts by weight of the hydrophobic elastomer, 70 to 150 parts by weight of the hydrocolloid particles, and 1 to 15 parts by weight of the aerogel particles.

3. The hydrocolloid composition according to claim 1, wherein the hydrophobic elastomer is a styrene-isoprene-styrene triblock copolymer (SIS).

4. The hydrocolloid composition according to claim 1, wherein the hydrocolloid particles are alginate crosslinked by calcium ions.

5. The hydrocolloid composition according to claim 1, wherein the aerogel particles are silica aerogel particles.

6. The hydrocolloid composition according to claim 1 or claim 5, wherein the aerogel particles have particles with hydrophobic functional groups formed on the particle surface.

7. The hydrocolloid composition according to claim 6, wherein the aerogel particles are hydrophobic aerogel particles, hybrid aerogel particles in which hydrophobic functional groups and hydrophilic functional groups are simultaneously formed on the particle surface, or a mixture of the hydrophobic aerogel particles, the hybrid aerogel particles and hydrophilic aerogel particles.

8. The hydrocolloid composition according to claim 1, further comprising a pressure-sensitive adhesive and/or a plasticizer.

9. A bio-patch comprising a hydrocolloid sheet having a hydrophobic elastomer matrix and hydrocolloid particles and aerogel particles dispersed within the matrix.

10. The bio-patch according to claim 9, further comprising:
an adhesive layer which is a pressure-sensitive adhesive layer disposed on the upper surface of the hydrocolloid sheet; and/or
a base layer that is a moisture-permeable and water-repellent film disposed on the lower surface of the hydrocolloid sheet.

11. The bio-patch according to claim 9, wherein the hydrocolloid sheet contains 100 parts by weight of the hydrophobic elastomer, 70 to 150 parts by weight of the hydrocolloid particles, and 1 to 15 parts by weight of the aerogel particles.

12. The bio-patch according to claim 9, wherein the hydrophobic elastomer is a styrene-isoprene-styrene triblock copolymer (SIS).

13. The bio-patch according to claim 9, wherein the hydrocolloid particles are alginate crosslinked by calcium ions.

14. The bio-patch according to claim 9, wherein the aerogel particles are silica aerogel particles.

15. The bio-patch according to claim 9 or claim 14, wherein the aerogel particles have particles with hydrophobic functional groups formed on the particle surface.

16. The bio-patch according to claim 15, wherein the aerogel particles are hydrophobic aerogel particles, hybrid aerogel particles in which hydrophobic functional groups and hydrophilic functional groups are simultaneously formed on the particle surface, or a mixture of the hydrophobic aerogel particles, the hybrid aerogel particles and hydrophilic aerogel particles.

17. The bio-patch according to claim 9, wherein the hydrocolloid sheet further comprises a pressure-sensitive adhesive and/or a plasticizer.
